(19) **Europäisches Patentamt**
**European Patent Office**
**Office européen des brevets**

(11) **EP 2 617 444 B1**

(12) **EUROPEAN PATENT SPECIFICATION**

(45) Date of publication and mention
of the grant of the patent:
**15.07.2020 Bulletin 2020/29**

(51) Int Cl.:
**A61M 1/36** *(2006.01)*     **A61M 1/34** *(2006.01)*

(21) Application number: **11825169.3**

(22) Date of filing: **13.09.2011**

(86) International application number:
**PCT/JP2011/070896**

(87) International publication number:
**WO 2012/036169 (22.03.2012 Gazette 2012/12)**

(54) **BLOOD PURIFICATION DEVICE AND CONTROL METHOD THEREFOR**

BLUTREINIGUNGSVORRICHTUNG UND STEUERVERFAHREN DAFÜR

DISPOSITIF DE PURIFICATION DE SANG ET SON PROCÉDÉ DE COMMANDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **15.09.2010 JP 2010206886**

(43) Date of publication of application:
**24.07.2013 Bulletin 2013/30**

(73) Proprietor: **Asahi Kasei Medical Co., Ltd.
Tokyo 100-0006 (JP)**

(72) Inventors:
• **NAMAZUDA Ken-ichiro
Tokyo 101-8101 (JP)**

• **KOBAYASHI Eisuke
Tokyo 101-8101 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(56) References cited:
WO-A1-79/01121     WO-A1-2010/073320
JP-A- 10 179 732    JP-A- 2001 087 381
JP-A- 2005 066 168   US-A- 4 191 182
US-A1- 2004 182 783   US-A1- 2005 182 349
US-A1- 2008 011 682

**Description**

**Technical Field**

The present invention relates to a blood purification device and further disclosed is a control method for the blood purification device.

**Background Art**

Currently, for treatment of intractable diseases such as autoimmune diseases and metabolic diseases, blood purification therapy is widely used. The blood purification therapy is roughly classified into therapeutic plasmapheresis and direct hemoperfusion (DHP). The therapeutic plasmapheresis is a treatment method for separating plasma components from blood taken from a patient and removing liquid factors that cause diseases (immune-related substances such as antibodies and inflammatory cytokines, metabolites such as LDL cholesterol, and toxic substances) and viruses, for example, from the plasma components to improve the patient's condition.

In the direct hemoperfusion (DHP) (Patent Literature 1), blood is directly treated, which makes it difficult to selectively remove specific pathogenic substances in plasma, and the application thereof is currently limited to therapy for specific diseases in which removal targets are cellular immune factors or toxic drugs, for example. In this regard, in the therapeutic plasmapheresis, plasma components are separated from blood and pathogenic substances are removed from the plasma components separated, whereby a wide variety of pathogenic substances in the plasma components can be selectively or non-selectively removed, and thus the applicable range of diseases is also wide and it is authorized that this method is covered by insurance as a therapy for various intractable diseases.

The therapeutic plasmapheresis herein mainly includes plasma exchange (PE), double filtration plasmapheresis (DFPP), and plasma adsorption (PA).

The plasma exchange (PE) is a therapy in which all plasma separated by a plasma separator is discarded and is replaced with the same amount of fresh frozen plasma. The performance of removing pathogenic substances in plasma components is high because all plasma components are discarded, but a large amount of plasma needs to be added, which presents a problem of side effects such as risk of infection or hypocalcemia.

In contrast, the double filtration plasmapheresis (DFPP) and the plasma adsorption (PA) are therapies in which plasma components separated by a plasma separator are treated by a plasma component separator or a plasma adsorber to remove pathogenic substances. In these therapies, a large portion of treated plasma components is returned to the patient without being discarded, whereby the amount of replacement fluid can be significantly reduced and further can be omitted, and side effects such as risk of infection or hypocalcemia can be reduced.

In the therapeutic plasmapheresis, catheters are widely used for vascular access. Examples of methods for vascular access include direct needle puncture using a needle inserted into an artery or a vein and catheterization. Catheterization is used in many facilities because the blood flow rate can be easily secured, long-term placement of a catheter is possible, and further it can be used for a plurality of applications such as securing a transfusion line when the therapeutic plasmapheresis is not performed. However, catheter insertion is performed exclusively by a doctor with specialized skill, operation and maintenance such as securing a catheter, cleaning the insertion site, and heparin lock are complicated, and further there is a problem that the invasiveness to a patient is high.

By contrast, direct needle punctuation with a needle is simple and the operation is easy, thereby enabling reduction of infection risk and invasiveness to a patient, but there is a disadvantage that the blood flow rate thus obtained is low compared with that in the case of the catheter.

In a blood purification device of Patent Literature 2, to solve problems such as clogging of a membrane or coagulation caused by reduction of the blood flow rate, a blood reservoir is provided to each of a blood removal-side circuit and a blood reinfusion-side circuit to increase the blood flow rate in a plasma separator. When the removed-blood flow rate of a patient is low with respect to the amount of fluid sent to the plasma separator and the amount of blood in the blood reservoir becomes depleted, by closing regular lines and opening a bypass circuit to store blood again in the blood reservoir while continuing circulation of blood to the plasma separator, the therapeutic plasmapheresis be performed under the condition that the removed-blood flow rate is continuously low.

In an apparatus for specific cell adsorption of Patent Literature 3, to improve adsorption selectivity of a specific cell under the condition of low removed-blood flow rates, a conduit for feeding blood in the outlet side of an adsorber back to the inlet side thereof and a feedback pump are provided, whereby the blood flow rate in the adsorber is increased.

## Citation List

Patent Literatures

[Patent Literature 1] Japanese Patent No. 4156160
[Patent Literature 2] Japanese Patent Application Laid-Open Publication No. 1987-246375
[Patent Literature 3] Japanese Patent Application Laid-Open Publication No. 2007-307280

US 4,191,182 discloses a process and apparatus for continuously separating blood into plasma and cellular component fractions and returning the latter to the subject in admixture with a makeup fluid.
US 2008/0011682 discloses a method for the depletion of at least one component of a fluid medium, with the concentration of the at least one component to be depleted being increased before its depletion. The fluid medium may be, for example, blood or blood plasma.

## Summary of Invention

### Technical Problem

However, the blood purification device of Patent Literature 2 requires time for storing blood in the reservoir, and thus extension of treatment time is unavoidable. This tendency becomes further noticeable particularly when closing the main lines and using the bypass circuit. Such extension of treatment time increases binding time for a patient, thereby significantly deteriorating his/her quality of life (QOL). In addition, two reservoirs have to be installed in the circuits, and thus there is a problem that physical load on the patient increases by increase of priming volume.
In the apparatus for specific cell adsorption of Patent Literature 3, direct hemoperfusion (DHP) is performed and a mechanism for separating plasma does not exist. In the direct hemoperfusion (DHP) for directly treating blood, various pathogenic substances in plasma components cannot be removed, and removal targets by this apparatus is limited to specific substances such as cellular immune factors in blood.
It is an object of the present invention to provide a blood purification device that improves patient's QOL and facilitates therapy management by setting the flow rate of blood taken from a subject in therapeutic plasmapheresis to the flow rate obtained in direct needle puncture with low invasiveness, also efficiently removes various pathogenic substances from plasma components, and can shorten the treatment time.

### Solution to Problem

As a result of exhaustive research to achieve the above-described object, the inventors of the present invention have found that in a blood purification device including a blood circuit that carries blood taken from a subject to a plasma separator through a blood removal-side circuit and returns blood passing through the plasma separator back to the subject through a blood reinfusion-side circuit, by providing a circulating circuit that connects the blood reinfusion-side circuit to the blood removal-side circuit and a circulating pump that circulates blood in the circulating circuit to the blood removal-side circuit, and circulating a portion of the blood flowing in the blood reinfusion-side circuit to the blood removal-side circuit in a state of pumping a portion of plasma components separated by the plasma separator, pathogenic substances can be efficiently removed from the plasma components while the flow rate of blood taken from the subject is set at a low flow rate, and also treatment time can be shortened.
More specifically, the present invention relates to a blood purification device comprising:

a blood circuit configured to carry blood taken from a subject to a plasma separator through a blood removal-side circuit and configured to return the blood passing through the plasma separator to the subject through a blood reinfusion-side circuit;
a blood pump configured to pump the blood in the blood circuit;
a circulating circuit configured to connect: the blood reinfusion-side circuit to the blood removal-side circuit;
a circulating pump configured to circulate the blood in the circulating circuit to the blood removal-side circuit;
a plasma circuit configured to carry plasma components separated by the plasma separator;
a plasma pump configured to pump the plasma components in the plasma circuit;
a control unit configured to control blood flow so that a portion of the blood flowing in the blood reinfusion-side circuit is circulated to the circulating circuit to flow to the blood removal-side circuit by driving the circulating pump when the blood in the blood circuit is being pumped and a portion of the plasma components separated by the plasma separator is being pumped to the plasma circuit by driving the blood pump and the plasma pump;

characterized in that the device further comprises a replacement fluid circuit configured to be connected to the blood reinfusion-side circuit and the replacement fluid circuit is connected upstream of a junction between the blood reinfusion-side circuit and the circulating circuit.

According to the present invention, by circulating a portion of the blood in the blood reinfusion-side circuit to the blood removal-side circuit, blood containing a high proportion of untreated plasma that has passed through the plasma separator can be circulated to the plasma separator and plasma separation can be performed again, whereby it becomes possible to increase the proportion of treated plasma. Accordingly, removal efficiency higher than that without this circulation can be obtained, whereby it becomes possible to significantly shorten the treatment time.

The plasma circuit of the blood purification device according to the present invention can also be characterized by including a plasma collection-side circuit that carries plasma components separated by the plasma separator to a plasma component separator and a plasma reinfusion-side circuit that returns plasma treated by the plasma component separator to the blood reinfusion-side circuit.

The blood purification device according to the present invention is characterized by further including a replacement fluid circuit that is connected to the blood reinfusion-side circuit, wherein the replacement fluid circuit is connected upstream of a junction between the blood reinfusion-side circuit and the circulating circuit.

The plasma reinfusion-side circuit of the blood purification device according to the present invention can also be characterized by being connected upstream of a junction between the blood reinfusion-side circuit and the circulating circuit.

The control unit of the blood purification device according to the present invention can also be characterized by including means for controlling drive of the blood pump, the circulating pump, and the plasma pump so that a flow rate of plasma components flowing in the plasma circuit becomes equal to or lower than 40% of sum total of a blood flow rate in the blood removal-side circuit and a circulation flow rate in the circulating circuit, and preferably equal to or lower than 30% thereof.

The control unit can also be characterized by including means for controlling drive of the blood pump so that the blood flow rate is 100 mL/min or less.

The control unit can also be characterized by including means for controlling drive of the blood pump so that the blood flow rate is 70 mL/min or less.

The control unit can also be characterized by including means for controlling drive of the blood pump so that the blood flow rate is 50 mL/min or less.

Also disclosed is a control method for a blood purification device including a blood circuit that carries blood taken from a subject to a plasma separator through a blood removal-side circuit and returns the blood passing through the plasma separator back to the subject through a blood reinfusion-side circuit; a blood pump that pumps the blood in the blood circuit; a circulating circuit that connects the blood reinfusion-side circuit to the blood removal-side circuit; a circulating pump that circulates the blood in the circulating circuit to the blood removal-side circuit; a plasma circuit that carries plasma components separated by the plasma separator; and a plasma pump that pumps the plasma components in the plasma circuit, wherein blood flow is controlled so that a portion of the blood flowing in the blood reinfusion-side circuit is circulated to the circulating circuit to be flown to the blood removal-side circuit by driving the circulating pump when the blood in the blood circuit is being pumped and a portion of the plasma components separated by the plasma separator is being pumped to the plasma circuit by driving the blood pump and the plasma pump.

According to the present disclosure , by circulating a portion of the blood in the blood reinfusion-side circuit to the blood removal-side circuit, blood containing a high proportion of untreated plasma that has passed through the plasma separator can be circulated to the plasma separator and plasma separation can be performed again, whereby it becomes possible to increase the proportion of treated plasma. Accordingly, removal efficiency higher than that without this circulation can be obtained, whereby it becomes possible to significantly shorten the treatment time.

The control method of the present disclosure for the blood purification device can also be one in which the plasma circuit includes a plasma collection-side circuit that carries plasma components separated by the plasma separator to a plasma component separator and a plasma reinfusion-side circuit that returns plasma treated by the plasma component separator to the blood reinfusion-side circuit.

The control method of the present disclosure for the blood purification device can also be one in which the plasma circuit includes a plasma collection-side circuit that carries plasma components separated by the plasma separator to a plasma component adsorber and a plasma reinfusion-side circuit that returns plasma treated by the plasma component adsorber to the blood reinfusion-side circuit.

The control method of the present disclosure for the blood purification device can also be one in which the blood purification device further includes a replacement fluid circuit that is connected to the blood reinfusion-side circuit and the replacement fluid circuit is connected upstream of a junction between the blood reinfusion-side circuit and the circulating circuit.

The control method of the present disclosure for the blood purification device can also be one in which the plasma reinfusion-side circuit is connected upstream of a junction between the blood reinfusion-side circuit and the circulating circuit.

Each control method of the present disclosure for the blood purification device described above can also be characterized in that drive of the blood pump, the circulating pump, and the plasma pump is controlled so that a flow rate of plasma components flowing in the plasma circuit becomes equal to or lower than 40% of sum total of a blood flow rate in the blood removal-side circuit and a circulation flow rate in the circulating circuit, and preferably equal to or lower than 30% thereof.

The control method of the present disclosure can also be characterized in that the blood pump is controlled so that the blood flow rate is 100 mL/min or less.

The control method of the present disclosure can also be characterized in that the blood pump is controlled so that the blood flow rate is 70 mL/min or less.

The control method of the present disclosure can also be characterized in that the blood pump is controlled so that the blood flow rate is 50 mL/min or less.

The control method according to the present disclosure can also be characterized by including a setting step of setting a treatment criterion, wherein a circulation flow rate necessary to maintain a pathogenic substance removal rate that is equivalent to or higher than the treatment criterion set is calculated from a removed-blood flow rate calculated from a drive rate of the blood pump and a plasma flow rate calculated from a drive rate of the plasma pump, and a drive rate of the circulating pump is controlled based on the circulation flow rate thus calculated.

The circulation flow rate can be calculated based on the following formula (1):
[Formula (1)]

$$Qr \geq \frac{-Q_b^2 + \dfrac{z}{ka(1-b)}AQ_b}{Q_b - A} \quad (1)$$

[in formula (1), $Q_b$ represents a removed-blood flow rate (mL/min), i.e., the amount of blood taken from a patient per unit time; $Q_f$ represents a plasma flow rate (mL/min), i.e., the amount of plasma separated by the plasma separator per unit time; $Q_r$ represents a circulation flow rate (mL/min), i.e., the amount of blood circulated from the blood reinfusion-side circuit to the blood removal-side circuit through the circulating circuit per unit time; The letter k represents a plasma collection ratio (-), i.e., the ratio of the flow rate of separated plasma to the flow rate of blood pumped to the plasma separator (sum of the removed-blood flow rate and the circulation flow rate); The letter a represents a permeation ratio (-) of pathogenic substances at the plasma separator; The letter b represents a purification ratio of pathogenic substances; The letter z represents plasma component fraction (%) in blood; The letter A represents a lower limit of the removed-blood flow rate (mL/min) necessary to reach the treatment criterion.]

**Advantageous Effects of Invention**

According to the present invention, by circulating a portion of blood in the blood reinfusion-side circuit to the blood removal-side circuit, blood containing a high proportion of untreated plasma that has passed through the plasma separator can be circulated to the plasma separator and plasma separation can be performed again, whereby it becomes possible to increase the proportion of treated plasma. Accordingly, removal efficiency higher than that without this circulation can be obtained, whereby it becomes possible to significantly shorten the treatment time. As a result of this, by direct needle puncture with low invasiveness, for example, treatment can be performed within a time equivalent to that required for catheterization with high blood flow rates.

**Brief Description of Drawings**

[Fig. 1] Fig. 1 is a diagram illustrating an outline of a blood purification device according to a first embodiment of the present invention.
[Fig. 2] Fig. 2 is a diagram illustrating an outline of a blood purification device according to a second embodiment of the present invention.
[Fig. 3] Fig. 3 is a diagram illustrating an outline of a blood purification device according to a third embodiment of the present disclosure.
[Fig. 4] Fig. 4 is a diagram illustrating an outline of a blood purification device according an example.
[Fig. 5] Fig. 5 is a diagram illustrating formulas for calculating treatment conditions that are necessary to meet one example of treatment criteria.
[Fig. 6] Fig. 6 is a diagram illustrating formulas for calculating treatment conditions that are necessary to meet another example of the treatment criteria.

**Description of Embodiments**

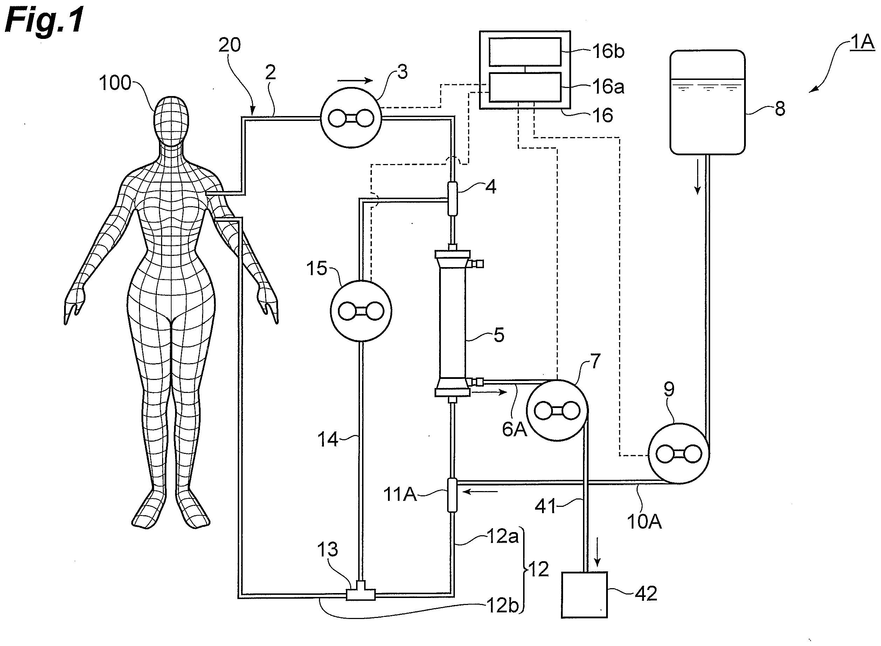

Preferred embodiments of the present invention will now be described with reference to the drawings. Fig. 1 is a diagram illustrating an outline of a blood purification device according to a first embodiment of the present invention.
In the following, any methods described which constitute methods for treatment of the human or animal body by surgery or therapy are not in accordance with the invention as claimed.

(First Embodiment)

As depicted in Fig. 1, a blood purification device 1A is used for plasma exchange (PE), for example. The blood purification device 1A a includes a blood circuit 20 that carries blood taken from a subject 100 to a plasma separator 5 through a blood removal-side circuit 2 and returns blood passing through the plasma separator 5 to the subject 100 through a blood reinfusion-side circuit 12, a blood pump 3 that pumps blood in the blood circuit 20, a circulating circuit 14 that connects the blood reinfusion-side circuit 12 to the blood removal-side circuit 2, a circulating pump 15 that circulates blood in the circulating circuit 14 to the blood removal-side circuit 2, a plasma circuit 6A that carries plasma components separated by the plasma separator 5, a plasma pump 7 that pumps the plasma components in the plasma circuit 6A, a waste fluid circuit 41 communicating with the plasma circuit 6A, and a waste fluid tank 42 connected to the waste fluid circuit 41. In the blood purification device 1A according to the first embodiment, plasma separated by the plasma separator 5 is pumped through the plasma circuit 6A and the waste fluid circuit 41 to the waste fluid tank 42 and is disposed therein.
In addition, the blood purification device 1A includes a replacement fluid circuit 10A that carries fluid for replacement to the blood reinfusion-side circuit 12, a replacement fluid pump 9 that pumps fluid in the replacement fluid circuit 10A, and a replacement fluid bag 8 that is connected to the upstream end of the replacement fluid circuit 10A and stores fluid for replacement. For convenience of explanation of the present embodiment (the same applies to each of the following embodiments), the upstream side of the blood reinfusion-side circuit 12 with respect to a connector (junction) 13 connected to the circulating circuit 14 is referred to as a blood reinfusion-side circuit upstream side 12a, and the downstream side thereof is referred to as a blood reinfusion-side circuit downstream side 12b. The replacement fluid circuit 10A is connected at any given position on the blood reinfusion-side circuit upstream side 12a with a connector, for example. In addition, the blood reinfusion-side circuit 12 is provided with a chamber 11A for preventing air or foreign matter, for example, mixed into blood from flowing into the body. The chamber 11A can be installed at any given position downstream of the junction between the replacement fluid circuit 10A and the blood reinfusion circuit 12. Note that the fluid for replacement is not particularly limited and, for example, blood derivatives in general can be used. Particularly in plasma exchange like the present embodiment, it is preferable to use fresh frozen plasma.
In addition, the blood purification device 1A includes a control unit 16 that controls the flow rate and path of blood. The control unit 16 includes a control board on which a CPU, a RAM, and a ROM, for example, are mounted, a memory, and a communication module that transmits and receives various control signals, for example, and implements various functions (e.g., flow rate control function) by the control board performing operation processing according to a predetermined program. The control unit 16 has blood pump drive control means 16a that is connected to the blood pump 3, the circulating pump 15, the plasma pump 7, the replacement fluid pump 9, and various valves not depicted by wired or wireless connections, and the blood pump drive control means 16a controls the flow rate and path of blood by drive-controlling the blood pump 3, the circulating pump 15, the plasma pump 7, and the replacement fluid pump 9. In addition, the control unit 16 includes a receiving unit 16b that receives predetermined input operations by a user. The receiving unit 16b is a touch panel, for example.
More specifically, the control unit 16 controls blood flow so that when blood in the blood circuit 20 is being pumped by driving the blood pump 3 and the plasma pump 7 and a portion of the plasma components separated by the plasma separator 5 is being pumped to the plasma circuit 6A, a portion of the blood flowing in the blood reinfusion-side circuit 12 is circulated to the circulating circuit 14 to be flown to the blood removal-side circuit 2 by driving the circulating pump 15.
A control method for the blood purification device 1A will be described hereinafter. During blood treatment, the blood pump 3 is controlled by the control unit 16, so that blood in the blood circuit 20 is pumped from the blood removal-side circuit 2 to the blood reinfusion-side circuit 12 via the plasma separator 5 at a predetermined flow rate. In addition, the plasma pump 7 is controlled, so that a portion of the plasma components separated by the plasma separator 5 is pumped to the plasma circuit 6A. Furthermore, the circulating pump 15 is controlled, so that a portion of the blood flowing in the blood reinfusion-side circuit 12 is circulated to the circulating circuit 14 and is returned to the blood removal-side circuit 2.

In addition, the control unit 16 controls drive of the blood pump 3, the circulating pump 15, and the plasma pump 7 so that the flow rate of plasma components (plasma flow rate) pumped to the plasma circuit 6A becomes equal to or lower than 40% of the sum total of the blood flow rate in the blood removal-side circuit 2 by drive of the blood pump 3 and the circulation flow rate in the circulating circuit 14 by drive of the circulating pump 15, and preferably equal to or lower than 30% thereof.

The control unit 16 can also control the blood pump 3 so that the flow rate of blood flowing in the blood removal-side circuit 2 is 100 mL/min or less. Furthermore, in terms of blood flow rate obtained in direct needle puncture with a needle, the blood flow rate is preferably 70 mL/min or less, and more preferably 50 mL/min or less. In addition, it is desirable in terms of efficiency of pathogenic substance removal that the flow rate of circulation flowing in the circulating circuit 14 be equal to or higher than 0 mL/min and the sum total of the blood flow rate and the circulation flow rate be equal to or lower than the maximum flow rate of blood flowing into the plasma separator 5.

Effects of the blood purification device 1A and the control method for the blood purification device 1A according to the present embodiments will be described hereinafter. For example, in a blood purification device that does not include the circulating circuit 14, the removal efficiency of plasma components is low, and thus treatment time becomes long for sufficient removal. On the contrary, the flow rate in the blood removal-side circuit needs to be increased to enable the treatment within a short time, and thus it is very difficult to solve both problems. Particularly in such a device used for plasma exchange, it is very difficult to shorten the treatment time because untreated plasma that is not separated by the plasma separator is generated.

However, with the blood purification device 1A and the control method for the blood purification device 1A, by circulating a portion of blood in the blood reinfusion-side circuit to the blood removal-side circuit, blood containing a high proportion of untreated plasma that is not separated by the plasma separator 5 can be circulated to the plasma separator 5 and plasma separation can be performed again, whereby the proportion of untreated plasma can be reduced. Accordingly, removal efficiency higher than that without this circulation can be obtained, whereby it becomes possible to significantly shorten the treatment time without excessively increasing the flow rate of removed blood (at a low flow rate). As a result of this, by direct needle puncture with low invasiveness, treatment can be performed within a time equivalent to that required for catheterization with high blood flow rates.

In addition, because the replacement fluid circuit 10A is connected upstream of the junction 13 between the blood reinfusion-side circuit 12 and the circulating circuit 14, it is possible to prevent the concentration (hematocrit) of blood cell components flowing in the circulating circuit 14 from becoming excessively high and, as a result of this, it is possible to continuously perform stable treatment while effectively preventing filtration failure associated with concentration of circulating blood.

(Second Embodiment)

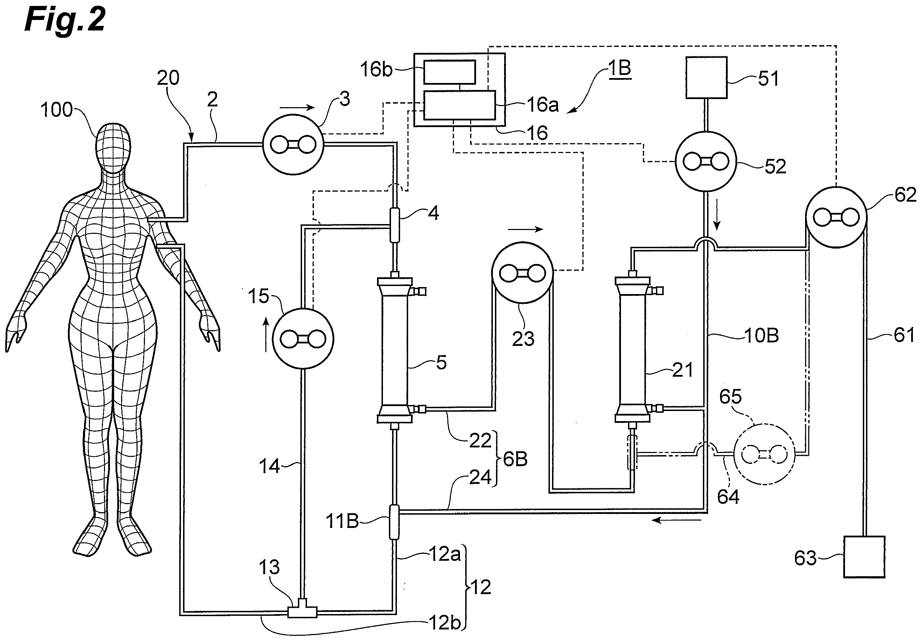

A blood purification device according to a second embodiment will now be described with reference to Fig. 2. Note that because this blood purification device 1B according to the second embodiment includes components and structures that are the same as those of the blood purification device 1A according to the first embodiment, an explanation will be made focusing on differences, the same reference numbers are given to substantially the same components and structures, and a detailed explanation thereof is omitted.

As depicted in Fig. 2, the blood purification device 1B is used for double filtration plasmapheresis (DFPP). The blood purification device 1B includes the blood circuit 20 having the blood removal-side circuit 2 and the blood reinfusion-side circuit 12, the plasma separator 5, the blood pump 3, the circulating circuit 14, and the circulating pump 15.

Furthermore, the blood purification device 1B includes a plasma circuit 6B that carries plasma components separated by the plasma separator 5. The plasma circuit 6B includes a plasma collection-side circuit 22 that carries plasma components separated by the plasma separator 5 to a plasma component separator 21, a plasma pump 23 that pumps plasma components in the plasma collection-side circuit 22 to the plasma component separator 21, and a plasma reinfusion-side circuit 24 that carries plasma components treated by the plasma component separator 21 to the blood reinfusion-side circuit 12.

Furthermore, the blood purification device 1B includes a waste fluid circuit 61 that carries plasma components passing through the plasma component separator 21, a waste fluid pump 62 that pumps plasma components in the waste fluid circuit 61, and a waste fluid tank 63 that stores the plasma components pumped by the waste fluid pump 62. The plasma components passing through the plasma component separator 21 are stored as a waste fluid in the waste fluid tank 63 and disposed.

In other words, the blood purification device 1B has the plasma collection-side circuit 22 that carries plasma components separated by the plasma separator 5 to a plasma component separator 21, the plasma reinfusion-side circuit 24 that returns plasma treated by the plasma component separator 21 to the blood reinfusion-side circuit 12, the waste fluid circuit (plasma waste fluid circuit) 61 that disposes a portion of plasma passing through the plasma component separator 21 without being treated, and the waste fluid pump (plasma waste fluid pump) 62 that pumps plasma in the plasma waste fluid circuit 61, and control using all of these components is performed in the blood purification device 1B. Alternatively, it is possible to perform control in such a state that the waste fluid pump (plasma waste fluid pump) 62 is not substantially driven and the waste fluid circuit (plasma waste fluid circuit) 61 is shut off, and performing such control is referred to as what is called dead-end filtration.

Furthermore, the blood purification device 1B includes a replacement fluid circuit 10B that carries fluid for replacement to the blood reinfusion-side circuit 12, a replacement fluid bag 51 that stores fluid for replacement, and a replacement fluid pump 52 that pumps fluid in the replacement fluid circuit 10B, and the replacement fluid circuit 10B is connected to the blood reinfusion-side circuit 12 via the plasma reinfusion-side circuit 24. Note that the fluid for replacement is not particularly limited and, for example, blood derivatives in general can be used. Particularly in double filtration plasmapheresis like the present embodiment, it is preferable to use albumin preparation.

The plasma reinfusion-side circuit 24 to which the replacement fluid circuit 10B is connected is connected at any given position on the blood reinfusion-side circuit upstream side 12a with a connector, for example. In addition, the blood reinfusion-side circuit 12 is provide with a chamber 11B for preventing air or foreign matter, for example, mixed into blood from flowing into the body. The chamber 11B can be installed at any given position downstream of the junction between the plasma reinfusion-side circuit 24 and the blood reinfusion circuit 12.

In addition, the control unit 16 of the blood purification device 1B is connected to the blood pump 3, the circulating pump 15, a plasma pump 23, the replacement fluid pump 52, the waste fluid pump 62 and various valves not depicted by wired or wireless connections, and controls the flow rate and path of blood by drive-controlling the blood pump 3, the circulating pump 15, and the plasma pump 23.

More specifically, the control unit 16 controls blood flow so that when blood in the blood circuit 20 is being pumped by driving the blood pump 3 and the plasma pump 23 and a portion of the plasma components separated by the plasma separator 5 is being pumped to the plasma circuit 6B, a portion of the blood flowing in the blood reinfusion-side circuit 12 is circulated to the circulating circuit 14 to be flown to the blood removal-side circuit 2 by driving the circulating pump 15.

A control method for the blood purification device 1B will be described hereinafter. During blood treatment, the blood pump 3 is controlled by the control unit 16, so that blood in the blood circuit 20 is pumped from the blood removal-side circuit 2 to the blood reinfusion-side circuit 12 via the plasma separator 5 at a predetermined flow rate. Furthermore, the circulating pump 15 is controlled, so that a portion of the blood flowing in the blood reinfusion-side circuit 12 is circulated to the circulating circuit 14 and returned to the blood removal-side circuit 2. In addition, the plasma pump 23 is controlled, so that a portion of the plasma components separated by the plasma separator 5 is pumped to the plasma component separator 21 through the plasma collection-side circuit 22. Plasma components treated by the plasma component separator 21 are pumped to the blood reinfusion-side circuit 12 through the plasma reinfusion-side circuit 24.

In addition, the control unit 16 controls drive of the blood pump 3, the circulating pump 15, and the plasma pump 23 so that the flow rate of plasma components (plasma flow rate) pumped to the plasma circuit 6B becomes equal to or lower than 40% of the sum total of the blood flow rate in the blood removal-side circuit 2 by drive of the blood pump 3 and the circulation flow rate in the circulating circuit 14 by drive of the circulating pump 15, and preferably equal to or lower than 30% thereof.

The control unit 16 can also control the blood pump 3 so that the flow rate of blood flowing in the blood removal-side circuit 2 is 100 mL/min or less. Furthermore, in terms of blood flow rate obtained in direct needle puncture with a needle, the blood flow rate is preferably 70 mL/min or less, and more preferably 50 mL/min or less. In addition, it is desirable in terms of efficiency of pathogenic substance removal that the flow rate of circulation flowing in the circulating circuit 14 be equal to or higher than 0 mL/min and the sum total of the blood flow rate and the circulation flow rate be equal to or lower than the maximum flow rate of blood flowing into the plasma separator 5.

With the blood purification device 1B and the control method for the blood purification device 1B according to the present embodiment, similarly to the blood purification device 1A and the control method for the blood purification device 1A, removal efficiency higher than that without this circulation can be obtained, whereby it becomes possible to significantly shorten the treatment time. As a result of this, by direct needle puncture with low invasiveness, treatment can be performed within a time equivalent to that required for catheterization with high blood flow rates.

In addition, in the plasma components separated from blood by the plasma separator 5, useful substances such as albumin are contained. With the blood purification device 1B according to the present embodiment, these useful substances can be selectively separated by the plasma component separator 21, and the useful substances thus separated can be returned to the blood reinfusion-side circuit 12 through the plasma reinfusion-side circuit 24.

In addition, because the plasma reinfusion-side circuit 24 is connected to the blood reinfusion-side circuit upstream side 12a of the blood reinfusion-side circuit 12 instead of the blood reinfusion-side circuit downstream side 12b thereof, it is possible to prevent the concentration (hematocrit) of blood cell components flowing in the circulating circuit 14 from becoming excessively high and, as a result of this, it is possible to continuously perform stable plasma separation while effectively preventing plasma separation failure in the plasma separator 5 associated with concentration of circulating blood.

Similarly, because the replacement fluid circuit 10B is connected upstream of the junction 13 between the blood reinfusion-side circuit 12 and the circulating circuit 14 via the plasma reinfusion-side circuit 24, it is possible to prevent the concentration (hematocrit) of blood cell components flowing in the circulating circuit 14 from becoming excessively high and, as a result of this, it is possible to continuously perform stable plasma separation while effectively preventing plasma separation failure in the plasma separator 5 associated with concentration of circulating blood. Note that in the present embodiment, the replacement fluid circuit 10B is connected to the blood reinfusion-side circuit 12 via the plasma reinfusion-side circuit 24, but may be connected directly to the blood reinfusion-side circuit 12 without intervention of the plasma reinfusion-side circuit 24.

As another control method for the blood purification device 1B, the waste fluid pump 62 may be stopped to appropriately shut off the upstream line of the waste fluid pump 62. In this case, substantially all plasma components that have passed through the plasma collection-side circuit 22 and been pumped to the plasma component separator 21 by the plasma pump 23 are treated with hollow fiber membranes and collected to the plasma reinfusion-side circuit 24. In other words, this control method is a control method for the blood purification device 1B that has the plasma collection-side circuit 22 that carries plasma components separated by the plasma separator 5 to the plasma component separator 21 and the plasma reinfusion-side circuit 24 that returns plasma treated by the plasma components separator 21 to the blood reinfusion-side circuit 12, and in which the waste fluid circuit 51 is substantially shut off.

The blood purification device 1B may also constitute a circulating circuit (hereinafter, referred to as a plasma circulation circuit) 64 (see two-dot chain lines in Fig. 2) by connecting the waste fluid circuit 61 to the plasma collection-side circuit 22. By carrying the plasma components that have passed through hollows of the hollow fiber membranes in the plasma component separator 21 and been pumped to the waste fluid circuit 61 to the plasma collection-side circuit 22 again without disposing them, it is possible to perform highly efficient plasma purification. Note that a connecting position of the waste fluid circuit 61 is preferred to be downstream of the plasma pump 23 in the plasma collection-side circuit 22. This control method may be a control method for a blood purification device that has the plasma collection-side circuit 22 that carries plasma components separated by the plasma separator 5 to the plasma component separator 21, the plasma reinfusion-side circuit 24 that returns plasma treated by the plasma component separator 21 to the blood reinfusion-side circuit 12, the plasma circulating circuit 64 that circulates a portion of plasma passing through the plasma component separator 21 without being treated to the plasma collection-side circuit 22, and the plasma circulating pump 65 that pumps plasma in the plasma circulating circuit. (Third Embodiment) (not according to the invention as claimed)

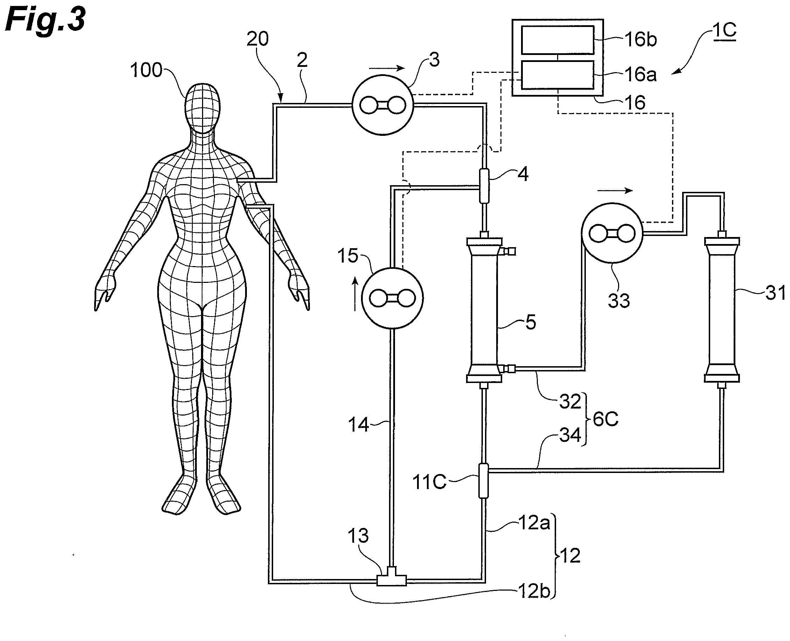

A blood purification device according to a third embodiment will now be described with reference to Fig. 3. Note that because this blood purification device 1C according to the third embodiment includes components and structures that are the same as those of the blood purification device 1A according to the first embodiment or the blood purification device 1B according to the second embodiment, an explanation will be made focusing on differences, the same reference numbers are given to substantially the same components and structures, and a detailed explanation thereof is omitted.

As depicted in Fig. 3, the blood purification device 1C is used for plasma adsorption (PA). The blood purification device 1C includes the blood circuit 20 having the blood removal-side circuit 2 and the blood reinfusion-side circuit 12, the plasma separator 5, the blood pump 3, the circulating circuit 14, and the circulating pump 15.

Furthermore, the blood purification device 1C include a plasma circuit 6C that carries plasma components separated by the plasma separator 5. The plasma circuit 6C includes a plasma collection-side circuit 32 that carries plasma components separated by the plasma separator 5 to a plasma component adsorber 31, a plasma pump 33 that pumps plasma components in the plasma collection-side circuit 32 to the plasma component adsorber 31, and a plasma reinfusion-side circuit 34 that carries plasma components treated by the plasma component adsorber 31 to the blood reinfusion-side circuit 12.

The blood reinfusion-side upstream side 12a of the blood reinfusion-side circuit 12 is provided with a chamber 11C that receives the plasma components treated by the plasma component adsorber 31, and the plasma reinfusion-side circuit 34 is connected to the chamber 11C. The chamber 11C can be installed at any given position downstream of the junction between the plasma reinfusion-side circuit 34 and the blood reinfusion-side circuit 12.

In addition, the control unit 16 of the blood purification device 1C is connected to the blood pump 3, the circulating pump 15, a plasma pump 33, and various valves not depicted by wired or wireless connections, and controls the flow rate and path of blood by drive-controlling the blood pump 3, the circulating pump 15, and the plasma pump 33 or open/close controlling the various valves.

More specifically, the control unit 16 controls blood flow so that when blood in the blood circuit 20 is being pumped by driving the blood pump 3 and the plasma pump 33 and a portion of the plasma components separated by the plasma separator 5 is being pumped to the plasma circuit 6C, a portion of the blood flowing in the blood reinfusion-side circuit 12 is circulated to the circulating circuit 14 to be flown to the blood removal-side circuit 2 by driving the circulating pump 15.

A control method for the blood purification device 1C will be described hereinafter. During blood treatment, the blood pump 3 is controlled by the control unit 16, so that blood in the blood circuit 20 is pumped from the blood removal-side circuit 2 to the blood reinfusion-side circuit 12 via the plasma separator 5 at a predetermined flow rate. Furthermore, the circulating pump 15 is controlled, so that a portion of the blood flowing in the blood reinfusion-side circuit 12 is circulated to the circulating circuit 14 and returned to the blood removal-side circuit 2. In addition, the plasma pump 33 is controlled, so that a portion of the plasma components separated by the plasma separator 5 is pumped to the plasma component adsorber 31 through the plasma collection-side circuit 32. Plasma components treated by the plasma component adsorber 31 are pumped to the blood reinfusion-side circuit 12 through the plasma reinfusion-side circuit 34.

In addition, the control unit 16 controls drive of the blood pump 3, the circulating pump 15, and the plasma pump 33 so that the flow rate of plasma components (plasma flow rate) pumped to the plasma circuit 6C becomes equal to or lower than 40% of the sum total of the blood flow rate in the blood removal-side circuit 2 by drive of the blood pump 3 and the circulation flow rate in the circulating circuit 14 by drive of the circulating pump 15, and preferably equal to or lower than 30% thereof.

The control unit 16 can also control the blood pump 3 so that the flow rate of blood flowing in the blood removal-side circuit 2 is 100 mL/min or less. Furthermore, in terms of blood flow rate obtained in direct needle puncture with a needle, the blood flow rate is preferably 70 mL/min or less, and more preferably 50 mL/min or less. In addition, it is desirable in terms of efficiency of pathogenic substance removal that the flow rate of circulation flowing in the circulating circuit 14 be equal to or higher than 0 mL/min and the sum total of the blood flow rate and the circulation flow rate be equal to or lower than the maximum flow rate of blood flowing into the plasma separator 5.

With the blood purification device 1C and the control method for the blood purification device 1C according to the present embodiment, similarly to the blood purification devices 1A and 1B and the control methods of the blood purification devices 1A and 1B, removal efficiency higher than that without this circulation can be obtained, whereby it becomes possible to significantly shorten the treatment time. As a result of this, by direct needle puncture with low invasiveness, treatment can be performed within a time equivalent to that required for catheterization with high blood flow rates.

The preferred embodiments of the present invention (First and Second Embodiments) and disclosure (Third Embodiment) have been described above with reference to Fig. 1, Fig. 2, and Fig. 3, but the present invention and disclosure is not limited to these embodiments.

(Fourth Aspect)

As a fourth aspect , still another control method that is executed in the above-described blood purification devices 1A to 1C will now be described. To begin with, the significance of this control method will be described.

In performing venipuncture using the above-described blood purification devices 1A to 1C, when the flow rate of blood flowing in the blood removal-side circuit 2 (removed-blood flow rate) is increased, the flow rate set cannot be obtained depending on conditions of blood flow of a patient, and accordingly the removed-blood flow rate is controlled to be as low as possible in some cases. In addition, increasing the flow rate of plasma components pumped to the plasma circuits 6A to 6C (plasma flow rate) leads to hemolysis of blood cell components in blood passing through the plasma separator 5, and accordingly the plasma flow rate is controlled to be as low as possible in some cases. In contrast, when the removed-blood flow rate and the plasma flow rate are controlled to be low, the pathogenic substance removal rate per unit time decreases. Accordingly, to achieve the pathogenic substance removal rate that is equivalent to or higher than a certain treatment criterion, it is necessary that the circulation flow rate in the circulating circuit 14 is controlled to be high.

However, because the optimal control range of the circulation flow rate changes depending on a decrease in each of the removed-blood flow rate and the plasma flow rate, when each of three flow rates of the removed-blood flow rate, the plasma flow rate, and the circulation flow rate is controlled solely, the circulation flow rate cannot be appropriately controlled depending on decreases in the removed-blood flow rate and the plasma flow rate, and consequently the treatment time may be extended, for example.

After exhaustive research to address the above-described new idea, the inventors of the present invention conceived a control method in which when a control system is controlling the removed-blood flow rate and the plasma flow rate at low flow rates, the circulation flow rate required for achieving the pathogenic substance removal rate that is equivalent to or higher than a predetermined treatment criterion can be calculated. This control method will be described below by taking a case where the blood purification device 1B according to the second embodiment as an example.

As depicted in Fig. 2, the blood purification device 1B includes the blood circuit 20 that carries blood taken from a subject 100 to the plasma separator 5 through a blood removal-side circuit 2 and returns blood passing through the plasma separator 5 to the subject through the blood reinfusion-side circuit 12, the blood pump 3 that pumps blood in the blood circuit 20, the circulating circuit 14 that connects the blood reinfusion-side circuit 12 to the blood removal-side circuit 2, the circulating pump 15 that circulates blood in the circulating circuit 14 to the blood removal-side circuit 2, the plasma circuit 6B that carries plasma components separated by the plasma separator 5, and the plasma pump 23 that pumps the plasma components in the plasma circuit 6B.

In addition, the blood purification device 1B includes the control unit 16 that controls the flow rate and path of blood. The control unit 16 includes the control board on which the CPU, the RAM, and the ROM, for example, are mounted, the memory, and the communication module that transmits and receives various control signals, for example, and implements various functions (e.g., flow rate control function) by the control board performing operation processing according to a predetermined program. The control unit 16 has the blood pump drive control means 16a, and the blood pump drive control means 16a controls the flow rate and path of blood by drive-controlling the blood pump 3, the circulating pump 15, the plasma pump 23, the replacement fluid pump 52, and the waste fluid pump 62. In addition, the control unit 16 includes the receiving unit 16b that receives predetermined input operations by a user.

The control method according to the present aspect includes a criterion setting step of setting a treatment criterion and a drive control step of calculating a circulation flow rate necessary to maintain a pathogenic substance removal rate that is equivalent to or higher than the treatment criteria set at the criterion setting step from the removed-blood flow rate calculated from a drive rate of the blood pump 3 and the plasma flow rate calculated from a drive rate of the plasma pump 23 and controlling a drive rate of the circulating pump 15 on the basis of the circulation flow rate thus calculated. Note that the setting of the treatment criterion is performed by using the receiving unit 16b of the control unit 16.

The circulation flow rate herein is calculated based on the following formula (1).

[Formula (1)]

$$Qr \geq \frac{-Q_b^{\,2} + \dfrac{z}{ka(1-b)} AQ_b}{Q_b - A} \qquad (1)$$

[In the formula (1), $Q_b$ represents the removed-blood flow rate (mL/min), i.e., the amount of blood taken from a patient per unit time. $Q_f$ represents the plasma flow rate (mL/min), i.e., the amount of plasma separated by the plasma separator per unit time. $Q_r$ represents the circulation flow rate (mL/min), i.e., the amount of blood circulated from the blood reinfusion-side circuit to the blood removal-side circuit through the circulating circuit. The letter k represents the plasma collection ratio (-), i.e., the ratio of the flow rate of separated plasma to the flow rate of blood pumped to the plasma separator (sum of the removed-blood flow rate and the circulation flow rate). The letter a represents the permeation ratio (-) of pathogenic substances at the plasma separator. The letter b represents the purification ratio of pathogenic substances in the plasma component separator. The letter z represents the plasma component fraction (%) in blood. The letter A represents the lower limit of the removed-blood flow rate (mL/min) necessary to reach the treatment criterion.]

A supplementary explanation for the above formula (1) is added herein. The "a", the permeation ratio of pathogenic substances in the plasma separator 5, is expressed by the following formula (2).

[Formula (2)]

$$a = C_f\,/\,C_{in} \qquad (2)$$

In the formula (2), "$C_{in}$" represents the concentration of pathogenic substances in plasma components in blood to be pumped to the plasma separator 5, and "Cf" represents the concentration of pathogenic substances in plasma components separated by the plasma separator 5. For example, when plasma components are separated by the plasma separator 5, if the concentration of pathogenic substances does not change, the result will be a=1 and, if no pathogenic substances are contained in the separated plasma components, the result will be a=0.

The "b", the purification ratio of pathogenic substances, is expressed by the following formula (3).

[Formula (3)]

$$b = C_p\,/\,C_f \qquad (3)$$

In the formula (3), "$C_f$" represents the concentration of pathogenic substances in plasma components separated by the plasma separator 5. "$C_p$" represents the concentration of pathogenic substances in plasma treated by the plasma component separator 21. For example, when plasma separated by the plasma separator 5 is pumped to the plasma component separator 21 and is treated therein, if no pathogenic substances are contained in the treated plasma, the result will be b=0 and, if pathogenic substances are not removed at all in the plasma component separator 21 and the concentration of pathogenic substances in the treated plasma does not change, the result will be b=1.

Note that to explain the present aspect taking the blood purification device 1B according to the above-described second embodiment as an example, the explanation has been made with "$C_p$" defined as the concentration of pathogenic substances in plasma treated by the plasma component separator 21. However, when executing the same control method using the blood purification device 1A according to the first embodiment, i.e., the control method including the criterion setting step of setting a treatment criterion and the drive control step of controlling the drive rate of the circulating pump 15 on the basis of the circulation flow rate calculated, "$C_p$" represents the concentration of pathogenic substances in replacement fluid. When executing the same control method using the blood purification device 1C (not according to the invention as claimed) according to the third embodiment, i.e., the control method including the criterion setting step of setting a treatment criterion and the drive control step of controlling the drive rate of the circulating pump 15 on the basis of the circulation flow rate calculated, "$C_p$" represents the concentration of pathogenic substances in plasma treated by the plasma component adsorber 31.

The "z", the plasma component fraction (%) in blood, is expressed by the following formula (4).
[Formula (4)]

$$z = \frac{100 - Hct}{100} \qquad (4)$$

Blood is composed of blood cell components including red blood cells and white blood cells, for example, and plasma components including water, electrolytes, and proteins, for example. The percentage of blood cell components in blood is generally referred to as hematocrit (Hct) (%), and inversely the percentage of plasma components in blood is defined herein as the plasma component fraction z.

The "A", the lower limit of the removed-blood flow rate necessary to reach the treatment criterion, will be described.

As one example of the treatment criterion, the case where a certain treatment condition is set as a comparative target will be described. When a treatment criterion is set such that the pathogenic substance removal rate equivalent to or higher than that under certain treatment conditions (reference removed-blood flow rate $Q_b{}^c$, reference circulation flow rate $Q_r{}^c$, reference plasma collection ratio $k^c$, reference plasma flow rate $Q_f{}^c$, reference treatment time $t^c$) is obtained, the removed-blood flow rate $Q_b$ needs to be higher than A calculated by the following formula (5) to meet the treatment criterion. The A calculated herein is the lower limit of the removed-blood flow rate required "when certain treatment conditions are set" as treatment criteria.
[Formula (5)]

$$A = \frac{\dfrac{k^c a(1-b)}{z - k^c a(1-b)} Q_b^c (Q_b^c + Q_r^c)}{\dfrac{z}{z - k^c a(a-b)} (Q_b^c + Q_r^c) - Q_r^c} \frac{t^c}{t} \qquad (5)$$

As an example, it is assumed hereinafter that the plasma separator 5 and the plasma component separator 21 whose permeation ratio and purification ratio of a certain pathogenic substance are respectively a=1 and b=0 for a patient with Hct 40 are used to perform double filtration plasmapheresis. When a treatment criterion is set such that "the pathogenic substance removal rate equivalent to or higher than that under certain treatment conditions (reference removed-blood flow rate $Q_b{}^c$=100 (mL/min), reference circulation flow rate $Q_r{}^c$=0 (mL/min), reference plasma collection ratio $k^c$=0.3, reference plasma flow rate $Q_f{}^c$=30 (mL/min), reference treatment time $t^c$=100 (min)) is obtained", the value of A is derived as in the following formula (6). More specifically, the removed-blood flow rate herein needs to satisfy $Q_b$>A=50 (mL/min).
[Formula (6)]

$$A = \frac{\dfrac{0.3 \times 1 \times (1-0)}{0.6 - 0.3 \times 1 \times (1-0)} \times 100 \times (100+0)}{\dfrac{0.6}{0.6 - 0.3 \times 1 \times (1-0)} \times (100+0) - 0} = 50 \qquad (6)$$

The case where patient blood flow volume (mL), a treatment time (min), and a pathogenic substance removal rate (%) are set as other treatment criteria will be described. The patient blood flow volume herein is the total amount of blood in the patient's body, and is represented by "P". The treatment time is represented by "t", and the pathogenic substance removal rate is represented by "d". The pathogenic substance removal rate is the ratio of pathogenic substances that can be removed from the patient's body by performing treatment for a predetermined time, and is calculated by the following formula (7), where $C_{before}$ is the concentration of pathogenic substances in the patient's plasma components before the treatment, and $C_{after}$ is the concentration of pathogenic substances in the patient's plasma components after the treatment.
[Formula (7)]

$$d = (1 - \frac{C_{after}}{C_{before}}) \times 100 \qquad (7)$$

When a treatment criterion is set such that "pathogenic substances can be removed by $d^c$ (%) for a certain patient with patient blood flow volume P (mL) within a treatment time $t^c$ (min)", the removed-blood flow rate $Q_b$ necessary to meet the treatment criterion needs to be higher than A calculated by the following formula (8). The A calculated herein is the lower limit of the removed-blood flow rate required "when the patient blood flow volume (mL), the treatment time (min), and the pathogenic substance removal rate (%) are set" as treatment criteria.
[Formula (8)]

$$A = \frac{P}{t} \ln(\frac{100}{100 - d^c}) \qquad (8)$$

As an example, it is assumed that the concentration of pathogenic substances is desired to be reduced to 50% by performing 100-minute treatment on a patient with a blood volume of 5000 milliliters. To meet this criterion without extending the treatment time, the removed-blood flow rate $Q_b$ needs to be larger than A calculated by the following formula (9). More specifically, the removed-blood flow rate in this case needs to satisfy $Q_b > A = 34.7$ (mL/min).
[Formula (10)]

$$A = \frac{5000}{100} \ln(\frac{100}{100 - 50}) \approx 34.7 \qquad (9)$$

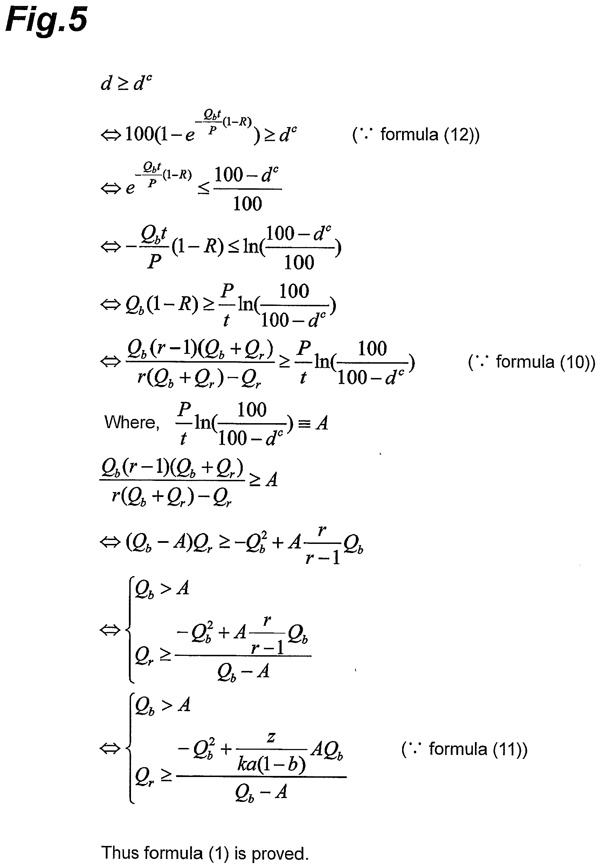

Referring to Fig. 5, the above-mentioned formula (1) that is used as a basis for calculating the circulation flow rate will be described hereinafter. To begin with, formula (10), formula (11), and formula (12) used in a derivation process are given.

Assuming that the concentration of pathogenic substances in plasma components in blood taken from a patient at a removed-blood flow rate $Q_b$ (mL/min) is $C_{in}$ (g/dL) and the concentration of pathogenic substances in plasma components in blood returned to the subject is $C_{out}$ (g/dL), the change ratio R (-) of the concentration of pathogenic substances during a process from taking blood to returning blood is expressed by the following formula (10).
[Formula (10)]

$$R = \frac{C_{out}}{C_{in}} = \frac{Q_b}{r(Q_b + Q_r) - Q_r} \qquad (10)$$

The "r" in the formula (10) is expressed by the following formula (11).
[Formula (11)]

$$r = \frac{z}{z - ka + kab} (\geq 1) \qquad (11)$$

In a one-compartment model, when blood purification treatment having the above-described removal performance is continued for a time t (min), the pathogenic substance removal rate d (%) can be expressed by the following formula (12).
[Formula (12)]

$$d = 100(1 - e^{-\frac{Q_b t}{P}(1-R)}) \qquad (12)$$

By using the above-mentioned formulas (10), (11), and (12), the formula (1) of inequality described above can be derived.

The case where two treatment criteria are set will be explained as an example, hereinafter. It is assumed that one of the treatment criteria is set such that that "the pathogenic substance removal rate equivalent to or higher than that under certain treatment conditions (reference removed-blood flow rate $Q_b{}^c$, reference circulation flow rate $Q_r{}^c$, reference plasma collection ratio $k^c$, reference plasma flow rate $Q_f{}^c$, reference treatment time $t^c$, reference pathogenic substance removal rate $d^c$) is obtained". In this case, treatment conditions (removed-blood flow rate $Q_b$, circulation flow rate $Q_r$, plasma collection ratio k, plasma flow rate $Q_f$, treatment time t, pathogenic substance removal rate d) necessary to meet this treatment criterion can be derived by formulas illustrated in Fig. 5.

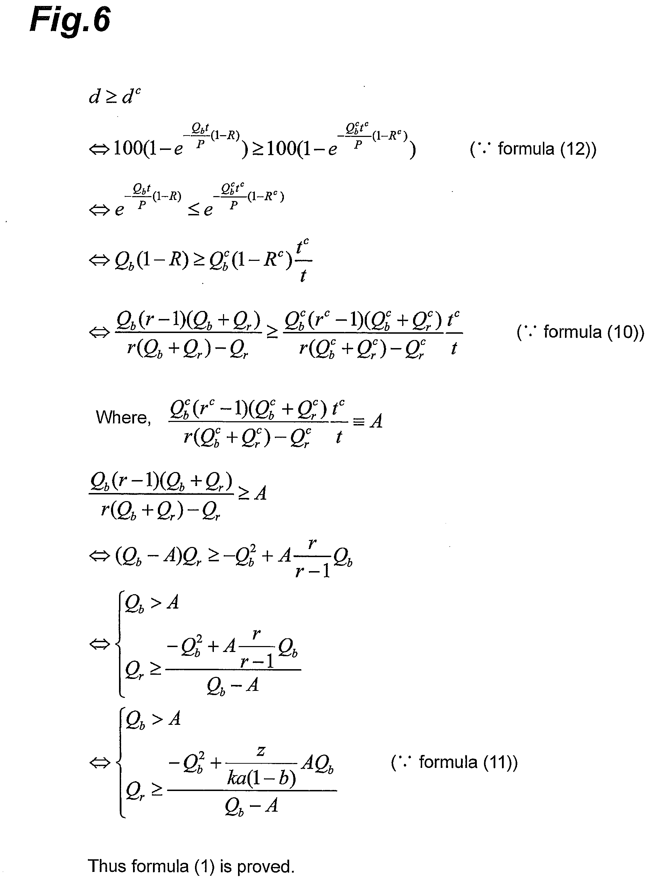

When the other of the treatment criteria is set such that "the pathogenic substance removal rate obtained by performing treatment on a certain patient with patient blood flow volume P (mL) for a treatment time $t^c$ (min) is $d^c$ (%)", treatment conditions (removed-blood flow rate $Q_b$, circulation flow rate $Q_r$, plasma collection ratio k, plasma flow rate $Q_f$, treatment time t, pathogenic substance removal rate d) necessary to meet this treatment criterion can be derived by formulas illustrated in Fig. 6.

The criterion setting step is performed by inputting the respective treatment criteria described above to the control unit 16. Furthermore, based on the formulas illustrated in Fig. 5 or Fig. 6, a circulation flow rate necessary to maintain the pathogenic substance removal rate equivalent to or higher than that under the treatment criteria set is calculated and, based on this circulation flow rate $Q_r$ thus calculated, the drive control step of controlling the drive rate of the circulating pump 15 is performed. As a result of this, even when the removed-blood flow rate and the plasma flow rate are controlled to be low, decrease of the pathogenic substance removal rate per unit time can be suppressed, and the pathogenic substance removal rate equivalent to or higher than a certain treatment criterion can be achieved.

**Examples**

To begin with, examples according to the second embodiment (Examples 1, 2, 3, and 4) and comparative examples (Comparative examples 1, 2, and 3) will be described.

(Example 1)

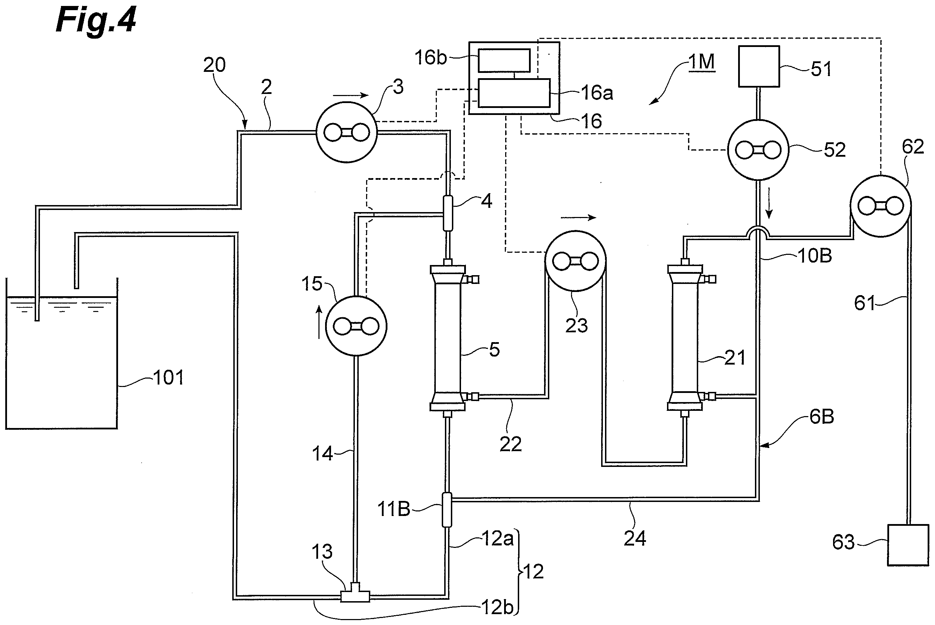

The examples will be described hereinafter with reference to Fig. 4. Note that the present invention is not limited to the examples. Fig. 4 is a diagram illustrating an outline of a blood purification device 1M according to the examples, and the same reference numbers are given to substantially the same components as those of the respective embodiments described above.

### (1) Fabrication of Plasma Separator

As the plasma separator 5, hollow fiber membranes that are polyethylene hollow fiber membranes having a inner diameter of 330 micrometers, a membrane thickness of 50 micrometers, and a pore size of 300 nanometers and coated with an ethylene-vinyl alcohol copolymer as a hydrophilic agent were used, these hollow fiber membrane were bundled together, and both ends of this bundle were secured to a polycarbonate container with polyurethane adhesive to fabricate a module. This module was used as the plasma separator 5 after being filled with saline and then being $\gamma$-ray sterilized. The effective membrane area per module was 0.8 m$^2$.

### (2) Fabrication of Plasma Component Separator

As the plasma component separator 21, hollow fiber membranes that are ethylene-vinyl alcohol copolymers having an inner diameter of 175 micrometers, a membrane thickness of 40 micrometers, and a pore size of 35 nanometers, these hollow fiber membranes were bundled together, and both ends of this bundle were secured to a polycarbonate container with polyurethane adhesive to fabricate a module. This module was used as the plasma component separator 21 after being filled with injection water and then being $\gamma$-ray sterilized. The effective membrane area per module was 2 m$^2$.

### (3) Fabrication of Blood Purification Device

As the blood purification device 1M, the plasma separator 5 and the plasma component separator 21 were connected by vinyl-chloride tubes, and vinyl-chloride tubes each having a portion squeezed by rollers were connected as the blood removal-side circuit 2, the circulating circuit 14, and the plasma collection-side circuit 22 as depicted in Fig. 4. A roller pump was used as the blood pump 3 after being connected to a squeezed portion of the blood removal-side circuit 2, a roller pump was used as the plasma pump 7 after being connected to a squeezed portion of the plasma collection-side circuit 22, and a roller pump was used as the circulating pump 15 after being connected to a squeezed portion of the circulating circuit 14.

### (4) Evaluation of LDL Cholesterol Removal Performance

Five liters of fresh bovine blood (T.P. $6.0\pm0.5$ g/dL, Hct $32\pm2\%$) was prepared. The fresh bovine blood heated at 37°C in a pool 101 was treated by the blood purification device 1M at a blood flow rate of 50 mL/min, a circulation flow rate of 50 mL/min, and a plasma flow rate of 30 mL/min (plasma collection ratio 0.3 (-)). Note that the replacement fluid pump and the waste fluid pump were not driven, and treatment in the plasma component separator 21 was performed in dead-end filtration mode. Blood in the pool 101 and blood at the outlet of the blood reinfusion-side circuit 12 were sampled at any given time, these blood samples were centrifuged at 3500 rpm for 10 minutes, and the LDL cholesterol concentrations of the supernatants thereof were measured. The permeation ratio for LDL cholesterol was calculated by the following formula (13).
[Formula (13)]

$$\text{Permeation ratio [-]} = \frac{\text{Concentration at outlet of blood reinfusion-side circuit}}{\text{Concentration in pool}} \qquad (13)$$

In addition, a time necessary for the LDL cholesterol concentration in the pool to be reduced to half of the initial concentration was defined as the treatment time. As a result, the permeation ratio for LDL cholesterol in one hour was 0.54 (-), and the treatment time was 137 minutes. Excellent removability for LDL cholesterol was obtained.

### (Example 2)

As depicted in Fig. 4, the blood purification device 1M that is the same as that of Example 1 was fabricated. By this blood purification device 1M, the fresh bovine blood heated at 37°C was treated at a blood flow rate of 70 mL/min, a circulation flow rate of 70 mL/min, and a plasma flow rate of 42 mL/min (plasma collection ratio 0.3 (-)). Blood in the pool 101 and blood at the outlet of the blood reinfusion-side circuit 12 were sampled at any given time, these blood samples were centrifuged at 3500 rpm for 10 minutes, and the LDL cholesterol concentrations of the supernatants thereof were measured. As a result, the permeation ratio for LDL cholesterol in one hour was 0.56 (-), and the treatment time was 112 minutes. Excellent removability for LDL cholesterol was obtained.

(Example 3)

As depicted in Fig. 4, the blood purification device 1M that is the same as that of Example 1 was fabricated. By this blood purification device 1M, the fresh bovine blood heated at 37°C was treated at a blood flow rate of 100 mL/min, a circulation flow rate of 100 mL/min, and a plasma flow rate of 60 mL/min (plasma collection ratio 0.3 (-)). Blood in the pool 101 and blood at the outlet of the blood reinfusion-side circuit were sampled at any given time, these blood samples were centrifuged at 3500 rpm for 10 minutes, and the LDL cholesterol concentrations of the supernatants thereof were measured. As a result, the permeation ratio for LDL cholesterol in one hour was 0.59 (-), and the treatment time was 63 minutes. Excellent removability for LDL cholesterol was obtained.

(Example 4)

As depicted in Fig. 4, the blood purification device 1M that is the same as that of Example 1 was fabricated. By this blood purification device 1M, the fresh bovine blood heated at 37°C was treated at a blood flow rate of 50 mL/min, a circulation flow rate of 100 mL/min, and a plasma flow rate of 45 mL/min (plasma collection ratio 0.3 (-)). Blood in the pool 101 and blood at the outlet of the blood reinfusion-side circuit 12 were sampled at any given time, these blood samples were centrifuged at 3500 rpm for 10 minutes, and the LDL cholesterol concentrations of the supernatants thereof were measured. As a result, the permeation ratio for LDL cholesterol in one hour was 0.40 (-), and the treatment time was 109 minutes. Excellent removability for LDL cholesterol was obtained. Compared with Example 1 using the same blood flow rate, the permeation ratio for LDL cholesterol was reduced by 0.14, and the treatment time was shortened by 28 minutes. By increasing the circulation flow rate, the permeation ratio can be reduced and the treatment time can be shortened further.

(Comparative Example 1)

A blood purification device that was the same as that of Example 1 except including no circulating circuit 14 was fabricated. By this blood purification device, the fresh bovine blood heated at 37°C was treated at a blood flow rate of 50 mL/min and a plasma flow rate of 15 mL/min (plasma collection ratio 0.3 (-)). Blood in the pool and blood at the outlet of the blood reinfusion-side circuit were sampled at any given time, these blood samples were centrifuged at 3500 rpm for 10 minutes, and the LDL cholesterol concentrations of the supernatants thereof were measured. As a result, the permeation ratio for LDL cholesterol in one hour was 0.67 (-), and the treatment time was 211 minutes. Compared with Example 1 using the same blood flow rate, the permeation ratio for LDL cholesterol was increased by 0.13, and the treatment time was extended by 74 minutes.

(Comparative Example 2)

The blood purification device that was the same as that of Example 1 except including no circulating circuit 14 was fabricated. By this blood purification device, the fresh bovine blood heated at 37°C was treated at a blood flow rate of 70 mL/min and a plasma flow rate of 21 mL/min (plasma collection ratio 0.3 (-)). Blood in the pool and blood at the outlet of the blood reinfusion-side circuit were sampled at any given time, these blood samples were centrifuged at 3500 rpm for 10 minutes, and the LDL cholesterol concentrations of the supernatants thereof were measured. As a result, the permeation ratio for LDL cholesterol in one hour was 0.70 (-), and the treatment time was 148 minutes. Compared with Example 2 using the same blood flow rate, the permeation ratio for LDL cholesterol was increased by 0.14, and the treatment time was extended by 36 minutes.

(Comparative Example 3)

The blood purification device that was the same as that of Example 1 except including no circulating circuit 14 was fabricated. By this blood purification device, the fresh bovine blood heated at 37°C was treated at a blood flow rate of 100 mL/min and a plasma flow rate of 30 mL/min (plasma collection ratio 0.3 (-)). Blood in the pool and blood at the outlet of the blood reinfusion-side circuit were sampled at any given time, these blood samples were centrifuged at 3500 rpm for 10 minutes, and the LDL cholesterol concentrations of the supernatants thereof were measured. As a result, the permeation ratio for LDL cholesterol in one hour was 0.73 (-), and the treatment time was 100 minutes. Compared with Example 3 using the same blood flow rate, the permeation ratio for LDL cholesterol was increased by 0.14, and the treatment time was extended by 37 minutes.

The above results are given in Table 1. By comparing Comparative Example 1 with Example 1 and Example 4, for example, it was verified that the permeation ratio for LDL cholesterol could be reduced and the treatment time could be shortened by circulating blood and further increasing the circulation flow rate even under conditions of the same blood flow rate.

[Table 1]

| | Example 1 | Example 2 | Example 3 | Example 4 | Comparative Example 1 | Comparative Example 2 | Comparative Example 3 |
|---|---|---|---|---|---|---|---|
| Blood flow rate $Q_b$ (mL/min) | 50 | 70 | 100 | 50 | 50 | 70 | 100 |
| Circulation flow rate $Q_r$ (mL/min) | 50 | 70 | 100 | 100 | 0 | 0 | 0 |
| Plasma flow rate $Q_f$ (mL/min) | 30 | 42 | 60 | 45 | 15 | 21 | 30 |
| LDL-C permeation ratio* (-) | 0.54 | 0.56 | 0.59 | 0.40 | 0.67 | 0.70 | 0.73 |
| Treatment time** (min) | 137 | 112 | 63 | 109 | 211 | 148 | 100 |
| *: each result is in one-hour value, **: time necessary for LDL removal rate to reach 50% | | | | | | | |

Examples according to the fourth aspect (Examples 5 and 6) and comparative examples (Comparative Examples 4 and 5) will be described hereinafter. On the examples according to the control method for the blood purification device including the criterion setting step and the drive control step and the comparative examples, verification was made with the blood purification device 1M that is the same as that of Example 1.

(Example 5)

Because (1) Fabrication of Plasma Separator, (2) Fabrication of Plasma Component Separator, and (3) Fabrication of Blood Purification Device herein are the same as those of Example 1, explanations thereof are omitted.

(4) Preparation of Subject

Five liters of fresh bovine blood (T.P. $6.0\pm0.5$ g/dL, Hct $32\pm2$%) was prepared.

(5) Settings of Treatment Criterion and Treatment Conditions

A pathogenic substance herein was assumed to be LDL cholesterol (a=1, b=0.15 (one-hour test values)). The treatment criterion was set such that "the pathogenic substance is removed by 50% by treatment on the subject (blood volume 5000 milliliters) for a treatment time of 120 minutes". To meet this treatment criterion, the blood flow rate $Q_b$ needs to be equal to or higher than a constant A that is calculated by the following formula (14). Therefore, $Q_b > 28.9$ (mL/min) needs to be satisfied.
[Formula (14)]

$$A = \frac{5000}{120}\ln(\frac{100}{100-50}) \approx 28.9 \qquad (14)$$

Assuming herein that the removed-blood flow rate $Q_b=50$ and the plasma collection ratio k=0.3, the condition of the circulation flow rate $Q_r$ is calculated as in the following formula (15) by using the above-described formula (1). Therefore, when the removed-blood flow rate $Q_b=50$, the circulation flow rate $Q_r > 64.1$, the plasma collection ratio k=0.3, the treatment criterion can be met.
[Formula (15)]

$$Qr \geq \frac{-50^2 + \dfrac{0.68}{0.3 \times 1 \times (1 - 0.15)} 28.9 \times 50}{50 - 28.9} = 64.1 \qquad (15)$$

(6) Evaluation of LDL Cholesterol Removal Performance

The fresh bovine blood heated at 37°C in the pool 101 was treated by the blood purification device 1M at the blood flow rate $Q_b$=50 mL/min, the circulation flow rate $Q_r$=100 mL/min, and the plasma collection ratio k=0.3. Note that the replacement fluid pump and the waste fluid pump were not driven, and treatment in the plasma component separator 21 was performed in dead-end filtration mode. Blood in the pool 101 was sampled at any given time, this blood sample was centrifuged at 3500 rpm for 10 minutes, and the LDL cholesterol concentration of the supernatant thereof was measured. A time necessary for the LDL cholesterol concentration in the pool to be reduced to half of the initial concentration was defined as the treatment time. As a result, the treatment time was 100 minutes. The circulation flow rate $Q_r$ set in Example 5 satisfies the condition (circulation flow rate $Q_r$>64.1) calculated by the formula (1), and it was verified in this case that the treatment criterion (that the LDL cholesterol concentration in the pool is reduced to half of the initial condition within 120 minutes) could be actually met.

As depicted in Fig. 4, the blood purification device 1M that is the same as that of Example 5 was fabricated. By this blood purification device 1M, the treatment was performed at the blood flow rate $Q_b$=50 mL/min, the circulation flow rate $Q_r$=75 mL/min, and the plasma collection ratio k=0.3. Note that the replacement fluid pump and the waste fluid pump were not driven, and treatment in the plasma component separator 21 was performed in dead-end filtration mode. Blood in the pool 101 was sampled at any given time, this blood sample was centrifuged at 3500 rpm for 10 minutes, and the LDL cholesterol concentration of the supernatant thereof was measured. As a result, the treatment time was 109 minutes. The circulation flow rate $Q_r$ set in Example 6 satisfies the condition (circulation flow rate $Q_r$>64.1) calculated by the formula (1), and it was verified in this case that the treatment criterion (that the LDL cholesterol concentration in the pool is reduced to half of the initial condition within 120 minutes) could be actually met.

(Comparative Example 4)

As depicted in Fig. 4, the blood purification device 1M that is the same as that of Example 5 was fabricated. By this blood purification device 1M, the treatment was performed at the blood flow rate $Q_b$=50 mL/min, the circulation flow rate $Q_r$=50 mL/min, and the plasma collection ratio k=0.3. Note that the replacement fluid pump and the waste fluid pump were not driven, and treatment in the plasma component separator 21 was performed in dead-end filtration mode. Blood in the pool 101 was sampled at any given time, this blood sample was centrifuged at 3500 rpm for 10 minutes, and the LDL cholesterol concentration of the supernatant thereof was measured. As a result, the treatment time was 131 minutes. The circulation flow rate $Q_r$ set in Comparative Example 4 does not satisfy the condition (circulation flow rate $Q_r$>64.1) calculated by the formula (1), and it was verified in this case that the treatment criterion (that the LDL cholesterol concentration in the pool is reduced to half of the initial condition within 120 minutes) could not be actually met.

(Comparative Example 5)

As depicted in Fig. 4, the blood purification device 1M that is the same as that of Example 5 was fabricated. By this blood purification device 1M, the treatment was performed at the blood flow rate $Q_b$=50 mL/min, the circulation flow rate $Q_r$=0 mL/min, and the plasma collection ratio k=0.3. Note that the replacement fluid pump and the waste fluid pump were not driven, and treatment in the plasma component separator 21 was performed in dead-end filtration mode. Blood in the pool 101 was sampled at any given time, this blood sample was centrifuged at 3500 rpm for 10 minutes, and the LDL cholesterol concentration of the supernatant thereof was measured. As a result, the treatment time was 215 minutes. The circulation flow rate $Q_r$ set in Comparative Example 5 does not satisfy the condition (circulation flow rate $Q_r$>64.1) calculated by the formula (1), and it was verified in this case that the treatment criterion (that the LDL cholesterol concentration in the pool is reduced to half of the initial condition within 120 minutes) could not be actually met.

The above-described results are given in Table 2. By comparing Example 5 with Comparative Example 5, for example, it was verified that the treatment criterion set in advance could actually be met when the treatment condition calculated by the formula (1) was satisfied.

[Table 2]

| | Example 5 | Example 6 | Comparative Example 4 | Comparative Example 5 |
|---|---|---|---|---|
| Blood flow rate $Q_b$ (mL/min) | 50 | 50 | 50 | 50 |
| Circulation flow rate $Q_r$ (mL/min) | 100 | 75 | 50 | 0 |
| Plasma flow rate $Q_f$ (mL/min) | 45 | 37.5 | 30 | 15 |
| Treatment time* (min) | 100 | 109 | 131 | 215 |
| *: time necessary for LDL removal rate to reach | | | | |

As described above, by the control method for the blood purification device 1M according to the present aspect , treatment effects that are equivalent to treatment criteria as targets can be achieved at different removed-blood flow rates $Q_b$, circulation flow rates $Q_r$, plasma flow rates $Q_f$ that are set in accordance with a state of a patient.

Therefore, even when the removed-blood flow rate cannot be sufficiently secured from an elderly patient, for example, or even when the plasma flow rate cannot be sufficiently secured from a patient at high risk for hemolysis, for example, by controlling pumps at the removed-blood flow rate $Q_b$, the circulation flow rate $Q_r$, and the plasma flow rate $Q_f$ for achieving treatment effects that are equivalent to the treatment criteria as targets, it is possible to provide stable treatment effect. In addition, the treatment time is a very important element for a patient to maintain a social life, and the control method in which treatment effects that are equivalent to treatment criteria as targets can be obtained by appropriately controlling the removed-blood flow rate $Q_b$, the circulating flow rate $Q_r$, and the plasma flow rate $Q_f$ under conditions in which the removed-blood flow rate $Q_b$ cannot be sufficiently secured provides high QOL for a patient.

## Industrial Applicability

With the blood purification device of the present invention and the control method therefor of the present disclosure, it is possible to obtain high removal efficiency of pathogenic substances. Accordingly, by direct needle puncture with low invasiveness, for example, treatment can be performed within a time equivalent to that required for catheterization with high blood flow rates. In addition, for a patient who is already catheterized, it is possible to shorten the treatment time or enhance treatment effects.

## Reference Signs List

1A, 1B, 1C, 1M... blood purification device, 2... blood removal-side circuit, 3... blood pump, 5... plasma separator, 6A, 6B, 6C... plasma circuit, 7, 23, 33... plasma pump, 8, 51... replacement fluid bag, 9, 52... replacement fluid pump, 10A, 10B... replacement fluid circuit, 12... blood reinfusion-side circuit, 12a... blood reinfusion-side circuit upstream side, 12b... blood reinfusion-side circuit downstream side, 13... connector (junction), 14... circulating circuit, 15 circulating pump, 16... control unit, 20... blood circuit, 21... plasma component separator, 22, 32... plasma collection-side circuit, 24, 34... plasma reinfusion-side circuit, 31... plasma component adsorber, 41, 61... waste fluid circuit, 62... waste fluid pump, 42, 63... waste fluid tank, 100... subj ect

## Claims

1. A blood purification device (1A/1B) comprising:

a blood circuit (20) configured to carry blood taken from a subject (100) to a plasma separator (5) through a blood removal-side circuit (2) and configured to return the blood passing through the plasma separator to the subject (100) through a blood reinfusion-side circuit (12);
a blood pump (3) configured to pump the blood in the blood circuit (20);
a circulating circuit (14) configured to connect the blood reinfusion-side circuit (12) to the blood removal-side circuit (2);
a circulating pump (15) configured to circulate the blood in the circulating circuit (14) to the blood removal-side circuit (2);
a plasma circuit (6A/6B) configured to carry plasma components separated by the plasma separator (5);
a plasma pump (7/23) configured to pump the plasma components in the plasma circuit (6A/6B);

a control unit (16) configured to control blood flow so that a portion of the blood flowing in the blood reinfusion-side circuit (12) is circulated to the circulating circuit (14) to flow to the blood removal-side circuit (2) by driving the circulating pump (15) when the blood in the blood circuit (20) is being pumped and a portion of the plasma components separated by the plasma separator (5) is being pumped to the plasma circuit (6A/6B) by driving the blood pump (3) and the plasma pump (7/23);

**characterized in that** the device (1A/1B) further comprises a replacement fluid circuit (10A/10B) configured to be connected to the blood reinfusion-side circuit (12) and the replacement fluid circuit (10A/10B) is connected upstream of a junction between the blood reinfusion-side circuit (12) and the circulating circuit (14).

**2.** The blood purification device (1A/1B) according to claim 1, wherein
the plasma circuit (6B) comprises a plasma collection-side circuit (22) configured to carry plasma components separated by the plasma separator (5) to a plasma component separator (21) and a plasma reinfusion-side circuit (24) configured to return plasma treated by the plasma component separator (21) to the blood reinfusion-side circuit (12).

**3.** The blood purification device (1B) according to claim 1, wherein
the plasma circuit (6B) comprises a plasma collection-side circuit (22) configured to carry plasma components separated by the plasma separator (5) to a plasma component separator (21) and a plasma reinfusion-side circuit (24) configured to return plasma treated by the plasma component separator (21) to the blood reinfusion-side circuit (12), and
the plasma reinfusion-side circuit (24) is connected upstream of a junction between the blood reinfusion-side circuit (12) and the circulating circuit (14).

**4.** The blood purification device (1A/1B) according to any one of claims 1 to 3, wherein
the control unit (16) comprises means for controlling drive of the blood pump (3), the circulating pump (15), and the plasma pump (7/23), and a flow rate of plasma components flowing in the plasma circuit (6A/6B) is controlled by the control unit (16) so as to be equal to or lower than 40 % of sum total of a blood flow rate in the blood removal-side circuit (2) and a circulation flow rate in the circulating circuit (14).

**5.** The blood purification device (1A/1B) according to claim 4, wherein
the control unit (16) comprises means for controlling drive of the blood pump (3), and the blood flow rate is controlled by the control unit (16) so as to be 100 mL/min or less.

**6.** The blood purification device (1A/1B) according to claim 4, wherein
the control unit (16) comprises means for controlling drive of the blood pump (3), and the blood flow rate is controlled by the control unit (16) so as to be 70 mL/min or less.

**7.** The blood purification device (1A/1B) according to claim 4, wherein
the control unit (16) comprises means for controlling drive of the blood pump (3), and the blood flow rate is controlled by the control unit (16) so as to be 50 mL/min or less.

**Patentansprüche**

**1.** Blutreinigungsvorrichtung (1A/1B), umfassend:

einen Blutkreislauf (20), der so konfiguriert ist, dass er von einem Patienten (100) entnommenes Blut über einen Kreislauf der Blutentnahmeseite (2) zu einem Plasmaseparator (5) transportieren kann, und so konfiguriert ist, dass er das durch den Plasmaseparator fließende Blut über einen Kreislauf der Blutreinfusionsseite (12) zu dem Patienten (100) zurückführen kann;
eine Blutpumpe (3), die so konfiguriert ist, dass sie das Blut in dem Blutkreislauf (20) pumpen kann;
einen Zirkulationskreislauf (14), der so konfiguriert ist, dass er den Kreislauf der Blutreinfusionsseite (12) mit dem Kreislauf der Blutentnahmeseite (2) verbindet;
eine Zirkulationspumpe (15), die so konfiguriert ist, dass sie das Blut in dem Zirkulationskreislauf (14) zu dem Kreislauf der Blutentnahmeseite (2) zirkulieren lassen kann;
einen Plasmakreislauf (6A/6B), der so konfiguriert ist, dass er durch den Plasmaseparator (5) abgetrennte Plasmakomponenten transportieren kann;
eine Plasmapumpe (7/23), die so konfiguriert ist, dass sie die Plasmakomponenten in dem Plasmakreislauf (6A/6B) pumpen kann;

eine Steuerungseinheit (16), die so konfiguriert ist, dass sie den Blutstrom so steuert, dass ein Teil des in dem Kreislauf der Blutreinfusionsseite (12) strömenden Bluts zum Zirkulationskreislauf (14) zirkulieren gelassen wird, um zum Kreislauf der Blutentnahmeseite (2) zu fließen, indem die Zirkulationspumpe (15) angetrieben wird, wenn das Blut in dem Blutkreislauf (20) gepumpt wird und ein Teil der durch den Plasmaseparator (5) abgetrennten Plasmakomponenten durch Antreiben der Blutpumpe (3) und der Plasmapumpe (7/23) zu dem Plasmakreislauf (6A/6B) gepumpt wird;

**dadurch gekennzeichnet, dass** die Vorrichtung (1A/1B) weiterhin einen Ersatzflüssigkeitskreislauf (10A/10B) umfasst, der so konfiguriert ist, dass er an den Kreislauf der Blutreinfusionsseite (12) angeschlossen werden kann, und der Ersatzflüssigkeitskreislauf (10A/10B) vor einer Verbindung zwischen dem Kreislauf der Blutreinfusionsseite (12) und dem Zirkulationskreislauf (14) angeschlossen wird.

2. Blutreinigungsvorrichtung (1A/1B) gemäß Anspruch 1, wobei der Plasmakreislauf (6B) einen Kreislauf der Plasmaaufnahmeseite (22), der so konfiguriert ist, dass er durch den Plasmaseparator (5) abgetrennte Plasmakomponenten zu einem Plasmakomponentenseparator (21) transportieren kann, und einen Kreislauf der Plasmareinfusionsseite (24), der so konfiguriert ist, dass er durch den Plasmakomponentenseparator (21) behandeltes Plasma zu dem Kreislauf der Blutreinfusionsseite (12) zurückführt, umfasst.

3. Blutreinigungsvorrichtung (1B) gemäß Anspruch 1, wobei der Plasmakreislauf (6B) einen Kreislauf der Plasmaaufnahmeseite (22), der so konfiguriert ist, dass er durch den Plasmaseparator (5) abgetrennte Plasmakomponenten zu einem Plasmakomponentenseparator (21) transportieren kann, und einen Kreislauf der Plasmareinfusionsseite (24), der so konfiguriert ist, dass er durch den Plasmakomponentenseparator (21) behandeltes Plasma zu dem Kreislauf der Blutreinfusionsseite (12) zurückführt, umfasst; und der Kreislauf der Plasmareinfusionsseite (24) vor einer Verbindung zwischen dem Kreislauf der Blutreinfusionsseite (12) und dem Zirkulationskreislauf (14) angeschlossen wird.

4. Blutreinigungsvorrichtung (1A/1B) gemäß einem der Ansprüche 1 bis 3, wobei die Steuerungseinheit (16) Einrichtungen zur Steuerung des Antriebs der Blutpumpe (3), der Zirkulationspumpe (15) und der Plasmapumpe (7/23) umfasst und die Strömungsgeschwindigkeit von Plasmakomponenten, die im Plasmakreislauf (6A/6B) fließen, durch die Steuerungseinheit (16) so gesteuert wird, dass sie kleiner oder gleich 40% der Summe der Blutströmungsgeschwindigkeit im Kreislauf der Blutentnahmeseite (2) und der Zirkulationsströmungsgeschwindigkeit im Zirkulationskreislauf (14) ist.

5. Blutreinigungsvorrichtung (1A/1B) gemäß Anspruch 4, wobei die Steuerungseinheit (16) Einrichtungen zur Steuerung des Antriebs der Blutpumpe (3) umfasst und die Blutströmungsgeschwindigkeit durch die Steuerungseinheit (16) so gesteuert wird, dass sie 100 ml/min oder weniger beträgt.

6. Blutreinigungsvorrichtung (1A/1B) gemäß Anspruch 4, wobei die Steuerungseinheit (16) Einrichtungen zur Steuerung des Antriebs der Blutpumpe (3) umfasst und die Blutströmungsgeschwindigkeit durch die Steuerungseinheit (16) so gesteuert wird, dass sie 70 ml/min oder weniger beträgt.

7. Blutreinigungsvorrichtung (1A/1B) gemäß Anspruch 4, wobei die Steuerungseinheit (16) Einrichtungen zur Steuerung des Antriebs der Blutpumpe (3) umfasst und die Blutströmungsgeschwindigkeit durch die Steuerungseinheit (16) so gesteuert wird, dass sie 50 ml/min oder weniger beträgt.

**Revendications**

1. Dispositif de purification de sang (1A/1B), comprenant :

un circuit sanguin (20) configuré pour transporter le sang prélevé chez un sujet (100) vers un séparateur de plasma (5) à travers un circuit latéral de prélèvement de sang (2), et configuré pour renvoyer le sang passant à travers le séparateur de plasma au sujet (100) par l'intermédiaire d'un circuit latéral de reperfusion de sang (12) ; une pompe à sang (3) configurée pour pomper le sang dans le circuit sanguin (20) ; un circuit de circulation (14) configuré pour connecter le circuit latéral de reperfusion de sang (12) au circuit latéral de prélèvement de sang (2) ; une pompe de circulation (15) configurée pour faire circuler le sang dans le circuit de circulation (14) vers le circuit latéral de prélèvement de sang (2) ;

un circuit à plasma (6A/6B) configuré pour transporter les composants plasmatiques séparés par le séparateur de plasma (5) ;

une pompe à plasma (7/23) configurée pour pomper les composants plasmatiques dans le circuit à plasma (6A/6B) ;

une unité de commande (16) configurée pour contrôler le flux sanguin de sorte qu'une partie du sang circulant dans le circuit latéral de reperfusion de sang (12) circule dans le circuit de circulation (14) pour s'écouler vers le circuit latéral de prélèvement de sang (2) en entrainant la pompe de circulation (15) lorsque le sang présent dans le circuit sanguin (20) est pompé, et qu'une partie des composants plasmatiques séparés par le séparateur de plasma (5) est pompée vers le circuit à plasma (6A/6B) en entrainant la pompe à sang (3) et la pompe à plasma (7/23) ;

**caractérisé en ce que** le dispositif (1A/1B) comprend en outre un circuit de fluide de substitution (10A/10B) configuré pour être connecté au circuit latéral de reperfusion de sang (12) et le circuit de fluide de substitution (10A/10B) est connecté en amont d'une jonction entre le circuit latéral de reperfusion de sang (12) et le circuit de circulation (14).

2. Dispositif de purification de sang (1A/1B) selon la revendication 1, dans lequel
le circuit à plasma (6B) comprend un circuit latéral de collecte de plasma (22) configuré pour transporter les composants plasmatiques séparés par le séparateur de plasma (5) vers un séparateur de composants plasmatiques (21), et un circuit latéral de reperfusion de plasma (24) configuré pour renvoyer le plasma traité par le séparateur de composants plasmatiques (21) vers le circuit latéral de reperfusion de sang (12).

3. Dispositif de purification de sang (1B) selon la revendication 1, dans lequel
le circuit à plasma (6B) comprend un circuit latéral de collecte de plasma (22) configuré pour transporter les composants plasmatiques séparés par le séparateur de plasma (5) vers un séparateur de composants plasmatiques (21), et un circuit latéral de reperfusion de plasma (24) configuré pour renvoyer le plasma traité par le séparateur de composants plasmatiques (21) vers le circuit latéral de reperfusion de sang (12), et
le circuit latéral de reperfusion de plasma (24) est connecté en amont d'une jonction entre le circuit latéral de reperfusion de sang (12) et le circuit de circulation (14).

4. Dispositif de purification de sang (1A/1B) selon l'une quelconque des revendications 1 à 3, dans lequel
l'unité de commande (16) comprend un moyen pour contrôler l'entrainement de la pompe à sang (3), de la pompe de circulation (15) et de la pompe à plasma (7/23), et un débit des composants plasmatiques circulant dans le circuit à plasma (6A/6B) est contrôlé par l'unité de commande (16) de manière à être inférieur ou égal à 40% de la somme totale d'un débit sanguin dans le circuit latéral de prélèvement de sang (2) et d'un débit de circulation dans le circuit de circulation (14).

5. Dispositif de purification de sang (1A/1B) selon la revendication 4, dans lequel
l'unité de commande (16) comprend un moyen pour contrôler l'entrainement de la pompe à sang (3), et le débit sanguin est contrôlé par l'unité de commande (16) pour qu'il soit inférieur ou égal à 100 mL/min.

6. Dispositif de purification de sang (1A/1B) selon la revendication 4, dans lequel
l'unité de commande (16) comprend un moyen pour contrôler l'entrainement de la pompe à sang (3), et le débit sanguin est contrôlé par l'unité de commande (16) pour qu'il soit inférieur ou égal à 70 mL/min.

7. Dispositif de purification de sang (1A/1B) selon la revendication 4, dans lequel
l'unité de commande (16) comprend un moyen pour contrôler l'entrainement de la pompe à sang (3), et le débit sanguin est contrôlé par l'unité de commande (16) pour qu'il soit inférieur ou égal à 50 mL/min.

**Fig.1**

EP 2 617 444 B1

Fig.2

Fig.3

Fig.4

# *Fig.5*

$$d \geq d^c$$

$$\Leftrightarrow 100(1 - e^{-\frac{Q_b t}{P}(1-R)}) \geq d^c \qquad (\because \text{ formula (12)})$$

$$\Leftrightarrow e^{-\frac{Q_b t}{P}(1-R)} \leq \frac{100 - d^c}{100}$$

$$\Leftrightarrow -\frac{Q_b t}{P}(1 - R) \leq \ln(\frac{100 - d^c}{100})$$

$$\Leftrightarrow Q_b(1 - R) \geq \frac{P}{t}\ln(\frac{100}{100 - d^c})$$

$$\Leftrightarrow \frac{Q_b(r - 1)(Q_b + Q_r)}{r(Q_b + Q_r) - Q_r} \geq \frac{P}{t}\ln(\frac{100}{100 - d^c}) \qquad (\because \text{ formula (10)})$$

Where, $\dfrac{P}{t}\ln(\dfrac{100}{100 - d^c}) \equiv A$

$$\frac{Q_b(r - 1)(Q_b + Q_r)}{r(Q_b + Q_r) - Q_r} \geq A$$

$$\Leftrightarrow (Q_b - A)Q_r \geq -Q_b^2 + A\frac{r}{r - 1}Q_b$$

$$\Leftrightarrow \begin{cases} Q_b > A \\ Q_r \geq \dfrac{-Q_b^2 + A\dfrac{r}{r - 1}Q_b}{Q_b - A} \end{cases}$$

$$\Leftrightarrow \begin{cases} Q_b > A \\ Q_r \geq \dfrac{-Q_b^2 + \dfrac{z}{ka(1 - b)}AQ_b}{Q_b - A} \end{cases} \qquad (\because \text{ formula (11)})$$

Thus formula (1) is proved.

# Fig.6

$$d \geq d^c$$

$$\Leftrightarrow 100(1 - e^{-\frac{Q_b t}{P}(1-R)}) \geq 100(1 - e^{-\frac{Q_b^c t^c}{P}(1-R^c)}) \qquad (\because \text{formula (12)})$$

$$\Leftrightarrow e^{-\frac{Q_b t}{P}(1-R)} \leq e^{-\frac{Q_b^c t^c}{P}(1-R^c)}$$

$$\Leftrightarrow Q_b(1 - R) \geq Q_b^c(1 - R^c)\frac{t^c}{t}$$

$$\Leftrightarrow \frac{Q_b(r-1)(Q_b + Q_r)}{r(Q_b + Q_r) - Q_r} \geq \frac{Q_b^c(r^c - 1)(Q_b^c + Q_r^c)}{r(Q_b^c + Q_r^c) - Q_r^c}\frac{t^c}{t} \qquad (\because \text{formula (10)})$$

Where, $\quad \dfrac{Q_b^c(r^c - 1)(Q_b^c + Q_r^c)}{r(Q_b^c + Q_r^c) - Q_r^c}\dfrac{t^c}{t} \equiv A$

$$\frac{Q_b(r-1)(Q_b + Q_r)}{r(Q_b + Q_r) - Q_r} \geq A$$

$$\Leftrightarrow (Q_b - A)Q_r \geq -Q_b^2 + A\frac{r}{r-1}Q_b$$

$$\Leftrightarrow \begin{cases} Q_b > A \\ Q_r \geq \dfrac{-Q_b^2 + A\dfrac{r}{r-1}Q_b}{Q_b - A} \end{cases}$$

$$\Leftrightarrow \begin{cases} Q_b > A \\ Q_r \geq \dfrac{-Q_b^2 + \dfrac{z}{ka(1-b)}AQ_b}{Q_b - A} \end{cases} \qquad (\because \text{formula (11)})$$

Thus formula (1) is proved.

28

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4156160 B
- JP 62246375 A
- JP 2007307280 A
- US 4191182 A
- US 20080011682 A